# EUROPÄISCHE PATENTSCHRIFT

(11) **EP 3 194 287 B1**
(45) Veröffentlichungstag und Bekanntmachung des Hinweises auf die Patenterteilung: **05.08.2020**
(21) Anmeldenummer: 15766749.4
(22) Anmeldetag: 15.09.2015
(51) Int. Cl.: B65D 43/02

(54) **BEHÄLTER MIT DECKEL**
CONTAINER WITH COVER
CONTENANT MUNI D'UN COUVERCLE

(30) Priorität: 15.09.2014 DE 102014013330
(43) Veröffentlichungstag der Anmeldung: 26.07.2017
(73) Patentinhaber: Mauser-Werke GmbH, 50321 Brühl (DE)
(72) Erfinder: BISCHOFF, Sebastian, 53859 Niederkassel (DE); SIEDE, Daniel, 50259 Pulheim (DE)
(74) Vertreter: Tilmann, Max Wilhelm
(86) Internationale Anmeldenummer: PCT/EP2015/001841
(87) Internationale Veröffentlichungsnummer: WO 2016/041632

(56) Entgegenhaltungen:
- EP-A1- 0 168 877
- EP-A2- 0 610 904
- NL-C2- 1 023 835
- US-A- 4 034 889
- US-A- 5 979 691

## Beschreibung

### Einleitung

Die Erfindung betrifft einen Behälter gemäß Anspruch 1.

### Stand der Technik

Behälter werden vielseitig eingesetzt und dienen insbesondere der Lagerung, dem Transport, der Sammlung und der Entsorgung von Stoffen oder Materialien, wobei Behälter in jeglicher Größe und Form bekannt sind. Abfallbehälter, die für den Einsatz in Krankenhäusern, Veterinär-Kliniken, Arzt- und Zahnarzt-Praxen sowie in medizinischen Forschungs- und Entwicklungslaboren vorgesehen sind, wobei insbesondere infektiöse Abfälle anfallen, werden nur einmalig gebraucht beziehungsweise mit den darin befindlichen Abfällen gemeinsam entsorgt, nachdem sie auf geeignete Weise dicht verschlossen wurden. Zum Verschließen werden typischerweise an dem Deckel befindliche Schnapphaken in dafür vorgesehene Rastöffnungen an dem Behälter eingeführt, wobei die Schnapphaken ihre Position beim Einrasten so gegenüber der Position beim Einführen ändern, dass sie ohne Manipulation nicht mehr aus der Rastöffnung entfernbar sind.

Ein derartiger Behälter mit Deckel und den eingangs genannten Merkmalen mit Ausnahme von Rastöffnungen in den Eckbereichen ist beispielsweise aus der EP 0 168 877 B1 bekannt, bei dem der Deckel so an dem Behälter fixiert wird, dass die Schnapphaken, die laschenartig mit endseitigem Hakenabschnitt ausgebildet sind und somit eine gewisse Elastizität in ihre Querrichtung aufweisen, in die Rastöffnungen eingeführt werden. Ist der Behälter mit dem Deckel verschlossen, greifen die Schnapphaken so in die Rastöffnungen ein, dass die Hakenabschnitte der Schnapphaken jeweils eine Wandung der Rastöffnungen umgreifen. Somit ist ein nachträgliches Entfernen des Deckels aufgrund der Vielzahl der am Umfang des Randflansches eingreifenden Schnapphaken kaum möglich. Lediglich durch ein gleichzeitiges Eindrücken aller Schnapphaken, so dass die Hakenabschnitte in die Ebene der Rastöffnungen gelangen, bei gleichzeitigem Anheben des Deckels kann selbiger entfernt werden, was sich jedoch selbst bei einem Mitwirken mehrerer Personen als äußerst schwierig erweist. Allerdings ist ein nachträgliches Öffnen eines mit dem Deckel verschlossenen Behälters aufgrund der Tatsache, dass die Behälter zur Lagerung und dem Abtransport sensibler Abfälle dienen, gar nicht erwünscht. Vielmehr ist ein dauernder Verschluss des Behälters bei dichtem Sitz des Deckels erforderlich, was ein gründliches Eingreifen der Schnapphaken in den Rastöffnungen bedingt.

Aus der US 5 979 691 ist ein weiterer Behälter mit Deckel und den Merkmalen des Oberbegriffs des Anspruchs 1 bekannt. Bei diesem Behälter sind auch in den Eckbereichen des Randflanschs gekrümmte Rastöffnungen angeordnet, wobei die zugehörigen Schnapphaken in den Eckbereichen des Deckels an die gekrümmten Rastöffnungen in den Eckbereichen des Randflanschs angepasst ausgebildet sind. Die Schnapphaken sind hier allerdings vergleichsweise lang und schmal ausgebildet, was ebenfalls zu einer gewissen Elastizität in ihrer Querrichtung führt.

Unerfreulicherweise kommt es in unzufrieden stellendem Maße vor, dass ein einzelner oder mehrere Schnapphaken - insbesondere solche der selben Reihe - beim Positionieren des Deckels entlang der Innenseite des Mantels gleiten anstatt in die dafür vorgesehenen Rastöffnungen im Randflansch einzugreifen, was dadurch bedingt ist, dass der Deckel nur schwer so auf den Behälter gedrückt werden kann, dass er horizontal ausgerichtet ist und alle Schnapphaken gleichzeitig in die Rastöffnungen gleiten. Dies ist deshalb schwierig, da die Schnapphaken aufgrund entstehender Reibkräfte und einer leichten Verbiegung derselben, beim Schließvorgang einen Widerstand erzeugen, der umso größer wird, je mehr Schnapphaken gleichzeitig einrasten sollen. Aus diesem Grund werden die Behälter typischerweise so mit einem Deckel verschlossen, indem der Deckel zunächst an einer Seite angesetzt wird, um dort die Schnapphaken einzuführen. Der somit schräg ausgerichtete und in einem Bereich bereits eingerastete Deckel wird dann sukzessive weiter auf dem Behälter fixiert, indem die Schnapphaken nacheinander in die dafür vorgesehenen Rastöffnungen eingeschoben werden, wobei sie oftmals kurzzeitig in eine Richtung quer zur Hochachse verbogen werden beziehungsweise eine leichte, bisweilen für den Nutzer unbemerkter Drehung des Deckels um seine Hochachse stattfindet. Dies ist deshalb möglich, da die laschenartigen Schnappnasen aufgrund ihrer Länge leicht in die vorgenannte Richtung federn können.

Hierbei kann es dazu kommen, dass bestimmte Schnapphaken dauerhaft an der inneren Mantelfläche anliegen (und somit keine Wirkung entfalten), wohingegen andere Schnapphaken ordnungsgemäß einrasten, so dass die Dichtigkeit des verschlossenen Behälters nicht gewährleistet ist, was insbesondere im Hinblick auf austretende Gerüche oder bei der Entsorgung des Behälters austretende Flüssigkeiten inakzeptabel ist. Dies gilt insbesondere, da der Transport potentiell infektiöser und ansteckender medizinischer Abfälle nach Transportrecht nur in vollumfänglich verschlossenen Behältern erlaubt ist. Ein Entfernen des Deckels zur neuen Positionierung des Deckels ist wegen der vorbeschriebenen quasi- irreversiblen Einrastung der übrigen Schnapphaken des Deckels nicht möglich.

### Aufgabe

Im Hinblick auf die vorgenannten Nachteile der bekannten Behälter ist es ist Aufgabe der vorliegenden Erfindung, einen Behälter mit den eingangs genannten Merkmalen derart weiterzuentwickeln, dass ein ordnungsgemäßes Eingreifen aller Schnapphaken in die dafür vorgesehenen Rastöffnungen gewährleistet ist.

### Lösung

Die vorstehende Aufgabe wird durch einen Behälter gemäß dem Anspruch 1 gelöst.

Dieser erfindungsgemäße Verlauf der Rastöffnungen, der im Nachfolgenden mit dem Wort "nicht-gerade" zusammengefasst wird, sorgt dafür, dass die zugehörigen Schnapphaken nicht in die Rastöffnungen gelangen können, wenn sie hierzu in Form einer Verdrehung um die Hochachse deformiert werden müssten, was bei normalem Krafteinsatz des Nutzers fast unmöglich ist. Während herkömmliche Schnapphaken, die parallel zu der Querachse oder der Längsachse verlaufen, eine gewisse Toleranz beim Einbringen in eine Rastöffnung mit sich bringen (insbesondere seitliche Auslenkungen quer zur Längsrichtung der zugeordneten Rastöffnung sind leicht möglich), kann der nicht-gerade verlaufende kompakte Schnapphaken nur bei ordnungsgemäßer Ausrichtung, d.h. auch ordnungsgemäßer Ausrichtung des Deckels insgesamt, in die von ihm vorgesehene Rastöffnung eingebracht werden, wenn er ordnungsgemäß eingeführt wird. Insbesondere würde ein Versuch, den nicht-geraden Schnapphaken in Querrichtung oder Längsrichtung etwas versetzt einführen zu wollen, scheitern, da dieser dann mit dem oberen Ende des Mantels kollidieren würde.

Darüber hinaus bewirken die erfindungsgemäß geformten Rastöffnungen, dass der Deckel nach Einführen des zugehörigen Schnapphakens in die gekrümmte, geneigte oder aus zwei Abschnitten zusammengesetzte Rastöffnung nicht mehr in Richtung der Längsachse oder Querachse verschoben werden kann, so dass erreicht wird, dass die übrigen Schnapphaken ausschließlich in die korrespondierenden Rastöffnungen gelangen können. Die mehreren "nicht-geraden" Schnapphaken übernehmen somit eine Führungsfunktion für das ordnungsgemäße Ausrichten und Einsetzen der übrigen, parallel zu der Längs- oder Querachse und vorzugsweise jeweils linear hintereinander angeordneten Schnapphaken, wie sie im Stand der Technik allein vorhanden sind. Eine Fehlschließung des Deckels, wie sie bei Behältern aus dem Stand der Technik vorkommen kann, ist somit (weitestgehend) ausgeschlossen.

Eine erfindungsgemäße gekrümmte Rastöffnung sowie ein korrespondierender Schnapphaken können demnach als Bogenabschnitt ohne Wendepunkt ausgeformt sein, oder aber eine kurvenartigen Verlauf mit Wendepunkt(en) aufweisen, wie beispielsweise einen S-förmigen Verlauf. Bei einer erfindungsgemäßen Rastöffnung bzw. bei einem erfindungsgemäßen Schnapphaken mit geradem Verlauf ist lediglich zu beachten, dass die Gerade eine Richtung ungleich der Querachse oder Längsachse des Behälters besitzt. Darüber hinaus ist jedoch auch denkbar, die Rastöffnungen sowie den zugehörigen Schnapphaken aus geraden Abschnitten zusammenzusetzen, die geneigt zueinander verlaufen, wobei beispielsweise zwei Abschnitte eine Ecke beliebigen Winkels oder aber auch mehrere gerade Abschnitte einen Teil eines Vielecks ausbilden können.

In einer vorteilhaften Ausgestaltung des erfindungsgemäßen Behälters besitzt der Mantel in zueinander parallelen Schnittebenen, die jeweils senkrecht zu der Hochachse des Behälters verlaufen, jeweils die Form eines abgerundeten Rechtecks, wobei vorzugsweise auch der Randflansch die Form eines abgerundeten Rechtecks besitzt und eine Mehrzahl von Rastöffnungen in paarweise gegenüberliegenden gerade verlaufenden Abschnitten des Randflanschs angeordnet sind und mindestens jeweils eine Rastöffnung in sämtlichen vier Bogenabschnitten des Randflanschs angeordnet ist, wobei die Bogenabschnitte jeweils zwei im rechten Winkel zueinander angeordnete geraden Abschnitte miteinander verbinden. Demnach bietet sich für die erfindungsgemäße Rastöffnung sowie für den zugehörigen Schnapphaken an, die Geometrie des Bogenabschnitts zu übernehmen, so dass Rastöffnung und Schnapphaken parallel zu einer Längsachse des Bogenabschnitts verlaufen.

Vorteilhafterweise sind die Schnapphaken jeweils aus einem dem Deckel zugewandten laschenartigen Zungenabschnitt und einem Hakenabschnitt zusammengesetzt, der jeweils an ein dem Deckel abgewandtes Ende des Zungenabschnitts anschließt, wobei vorzugsweise die Zungenabschnitte in eine Richtung senkrecht zu der Hochachse des Behälters elastisch verformbar sind.

Im Hinblick auf ein einfaches Aufsetzen des Deckels ist es besonders von Vorteil, wenn die Rastöffnungen jeweils auf ihren der Hochachse des Behälters zugewandten Seiten einen Abstand zu dem Mantel aufweisen, der kleiner als 5 mm, vorzugsweise kleiner als 2 mm, weiter vorzugsweise kleiner als 1 mm, noch weiter vorzugsweise gleich Null ist. Je geringer der Abstand der Rastöffnungen zu dem Mantel ist, desto komfortabler lassen sich die Schnapphaken des Deckels in die Rastöffnungen einführen.

Dem Boden abgewandtes Ende des Behälters wird von einem umlaufenden Dichtsteg gebildet, der vorzugsweise eine Verlängerung einer umlaufenden, den Mantel definierenden Behälterwandung bildet, wobei der Dichtsteg in der Schließstellung des Behälters dichtend an einer Dichtung anliegt, die sich in einer an den Dichtsteg angepassten umlaufenden Dichtnut in dem Deckel befindet, wobei sich ein Endabschnitt des Dichtstegs vorzugsweise in die Dichtnut hin erstreckt, zeichnet sich der geschlossene Behälter durch seine Dichtigkeit aus, die unter anderem auch hinsichtlich in dem Behälter entstehender Gerüche vorteilhaft ist. Dabei ist es besonders von Vorteil, wenn der umlaufende Dichtsteg auch dann noch an der Dichtung in der Dichtnut anliegt, wenn der Behälter an einem an dem Deckel vorgesehenen Haltegriff angehoben wird und somit eine Relativverschiebung zwischen Deckel und Behälter erfolgen kann, ohne die Dichtigkeit der Verbindung zu beeinträchtigen.

Schließlich sei angemerkt, dass die verschiedenen Merkmale der Unteransprüche je einzeln für sich oder zu mehreren in beliebigen Kombinationen bei Varianten der Erfindung verwirklicht sein können.

### Ausführungsbeispiel:

Die vorstehend beschriebene Erfindung wird nachfolgend anhand von Ausführungsbeispielen, die in den Figuren dargestellt sind, näher erläutert.

Es zeigt:
- Figuren 1a und 1b:: dreidimensionale Ansichten eines erfindungsgemäßen Behälters,
- Figuren 2a bis 2c:: eine Frontansicht, eine Unteransicht sowie eine Draufsicht des Behälters aus den Figuren 1,
- Figur 3:: eine Draufsicht auf den Behälter aus Figur 1 ohne Deckel,
- Figuren 4a bis 4f:: dreidimensionale und zweidimensionale Ansichten des Deckels aus Figur 1 und
- Figur 5:: einen Vertikalschnitt durch den Deckel und den Behälter im Bereich eines Schnapphakens und einer Rastöffnung.

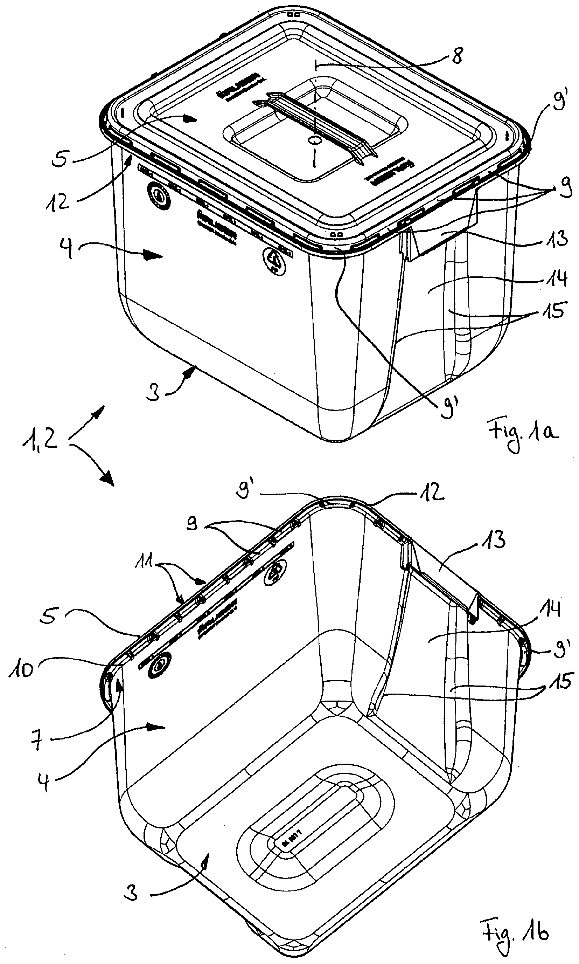

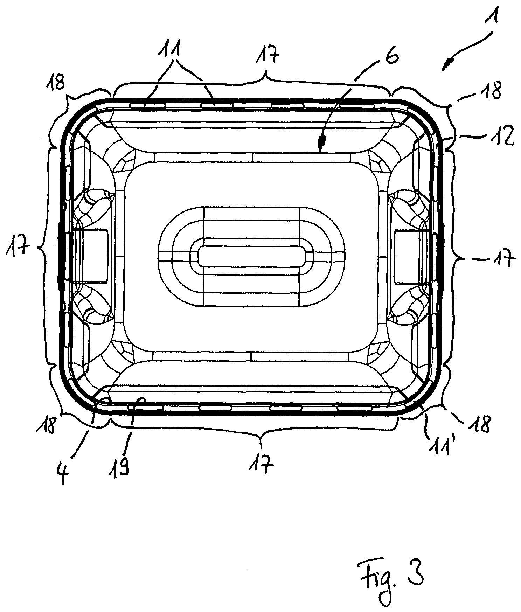

Eine Ausführungsform für einen erfindungsgemäßen Behälter 1 ist in den Figur 1a und 1b dargestellt, die eine dreidimensionale Ansicht des Behälters 1 in seiner Schließstellung 2 von oben beziehungsweise von unten zeigt. Der Behälter 1 setzt sich aus einem Boden 3, einem daran umlaufend anschließenden Mantel 4 und einem Deckel 5 zusammen, wobei der Deckel 5 einen Öffnungsquerschnitt 6 (siehe Figur 3) zu einem Innenvolumen des Behälters 1 verschließt, der von einem dem Boden 3 abgewandten Ende 7 des Mantels 4 definiert wird. Eine Hochachse 8 des Behälters 1 fällt mit der Vertikalen zusammen. In der gezeigten Schließstellung 2 des Behälters 1 greifen Schnapphaken 9, 9', die an Rändern 10 des Deckels 5 umlaufend verteilt und jeweils beabstandet zueinander angeordnet sind, in korrespondierende Rastöffnungen 11, 11' eines umlaufend nach außen vorstehenden Randflansches 12 des Mantels 4 ein. Die Anordnung und Geometrie des Randflansches 12 mit seinen Rastöffnungen 11, 11' sowie der Schnapphaken 9, 9' des Deckels 5 wird in den nachfolgenden Figuren besser deutlich.

An sich gegenüberliegenden kurzen Seiten ist der Behälter 1 an einer Oberseite jeweils mit einer Tragelasche 13 ausgestattet. Um einen komfortablen Eingriff in die Tragelaschen 13 zu ermöglichen und die Stabilität des Behälters 1 im Bereich der Tragelaschen 13 zu erhöhen, weist dieser an seinen kurzen Seitenflächen eine vertikal verlaufende Einbuchtung 14 auf, deren Breite an der Oberseite des Behälters 1 in etwa einer Länge der Tragelasche 13 entspricht, wohingegen deren Breite am Boden 3 des Behälters 1 größer ist. Folglich verlaufen zwei sich gegenüberliegende Rippen 15 der Einbuchtung 14 schräg zueinander.

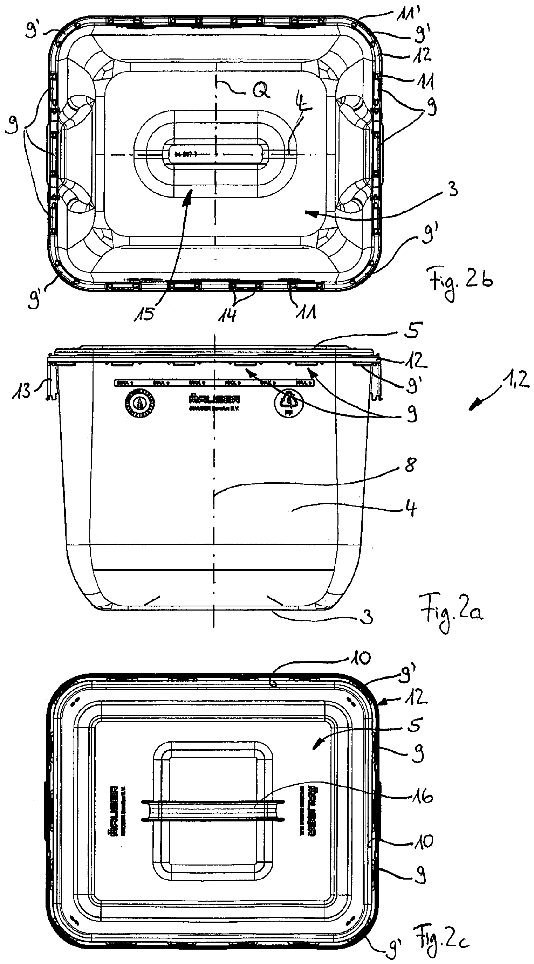

Der Mantel 4 des Behälters 1 mit seinem Randflansch 12 sowie auch der Deckel 5 weisen jeweils in allen Horizontalschnitten die Form eines abgerundeten Rechtecks auf, was auch aus den Figuren 2a bis 2c gut erkennbar ist.

Die Figur 2a zeigt eine Frontansicht des Behälters 1 auf eine lange Seitenfläche desselben, aus der deutlich wird, dass die Hochachse 8 senkrecht zum Boden 3 des Behälters 1 verläuft. Der Randflansch 12 wird in nicht in der Figur 2a erkennbaren Rastöffnungen 11, 11' von den Schnapphaken 9, 9' des Deckels 5 durchdrungen, so dass eine irreversible, dichte Schließung des Behälters 1 erreicht wird.

Aus der Figur 2b, die eine Unteransicht des Behälters 1 zeigt, ist der Randflansch 12 mit seinen umlaufend verteilten und jeweils zueinander beabstandeten Rastöffnungen 11, 11' erkennbar, wobei sich jeweils in den Rastöffnungen 11, 11' die eingreifenden Schnapphaken 9, 9' befinden. In Längsrichtung der Rastöffnungen 11, 11' und der Schnapphaken 9, 9' betrachtet, die jeweils in Umfangsrichtung des Randflansches 12 verläuft, weisen die Rastöffnungen 11, 11' eine größere Länge auf als die Schnapphaken 9, 9', so dass neben den Schnapphaken 9, 9' zu beiden Richtungen kleine Zwischenräume 14 zu Querwandungen der Rastöffnungen 11, 11' verbleiben. Die Rastöffnungen 11, 11' werden unterschieden in gerade ausgebildete Rastöffnungen 11 und gebogen ausgebildete Rastöffnungen 11'. Analog dazu gibt es gerade verlaufende Schnapphaken 9 und gebogene Schnapphaken 9', wobei sich die gebogenen Rastöffnungen 11' und Schnapphaken 9' in den vier abgerundeten Ecken des Randflansches 12 und des Deckels 5 befinden. Somit verlaufen die Rastöffnungen 11' und die Schnapphaken 9' insgesamt gekrümmt und geneigt zu einer Querachse Q und eine Längsachse L des Behälters 1.

Ebenfalls ist der Figur 2b zu entnehmen, dass der Boden 3 des Behälters 1 eine zentral angeordnete Vertiefung 15 aufweist, die in ihren Abmessungen einem Haltegriff 16 am Deckel 5, der in Figur 2c zu sehen ist, angepasst ist.

Der Figur 2c, die eine Draufsicht des mit dem Deckel 5 verschlossenen Behälters 1 zeigt, sind ebenfalls der Randflansch 12, die Ränder 10 des Deckels 5, die Schnapphaken 9, 9' und die Rastöffnungen 11, 11' zu entnehmen. Darüber hinaus ist der Haltegriff 16 in einem zentralen Bereich des Deckels 5 gezeigt.

Um deutlicher auf die Geometrie der Rastöffnungen 11, 11' eingehen zu können, zeigt die Figur 3 eine Draufsicht auf den Behälter 1 ohne Deckel 5. Zum einen weist der Randflansch 12 an gerade verlaufenden Abschnitten 17 eine Mehrzahl von paarweise gegenüberliegenden Rastöffnungen 11 auf, die langgestreckt ausgebildet sind und ebenfalls einen geraden Verlauf besitzen. Entlang der langen Abschnitte 17 des Randflansches 12 sind jeweils vier Rastöffnungen 11 und entlang der kurzen Abschnitte 17 jeweils nur drei Rastöffnungen 11 vorgesehen. Aufgrund seiner Form als abgerundetes Rechteck umfasst der Randflansch 12 ferner vier Bogenabschnitte 18, die jeweils zwei senkrecht zueinander verlaufende gerade Abschnitte 17 miteinander verbinden, also jeweils einen kurzen geraden mit einem langen geraden Abschnitt 17. In den Bogenabschnitten 18 befinden sich jeweils auch eine Rastöffnung 11', die analog zu der Biegung des Bogenabschnitts 18 gebogen verlaufen. Eine der Hochachse 8 zugewandte Seite 19 der Rastöffnungen 11, 11' weist jeweils keinen Abstand zu dem Mantel 4 auf, was bedeutet, dass die der Hochachse 8 zugewandte Seite 19 der Rastöffnungen 11, 11' mit dem Mantel 4 fluchtet.

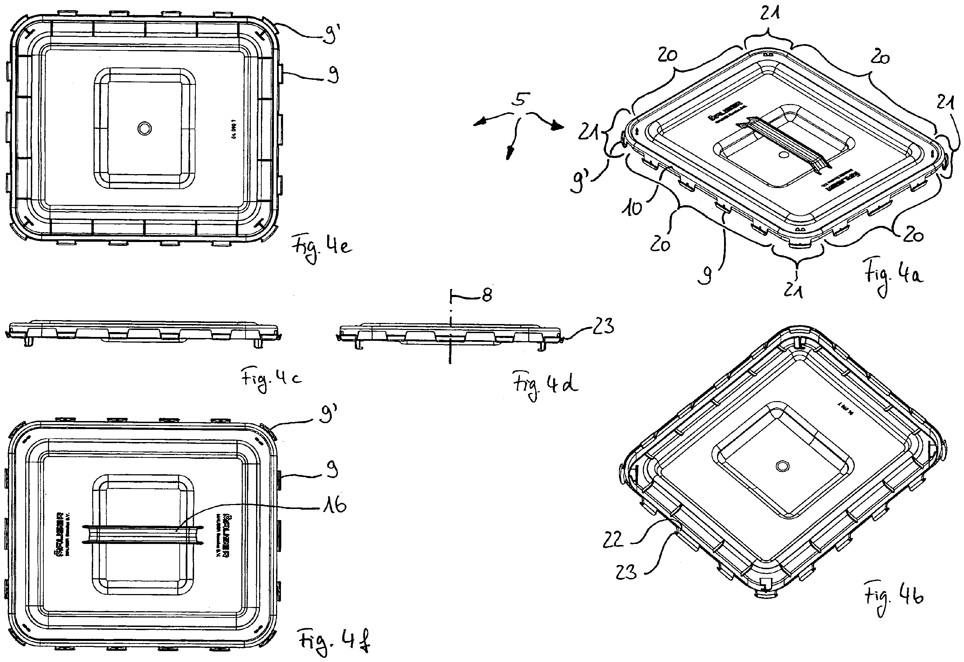

Die Figuren 4a bis 4f bilden lediglich den Deckel 5 aus verschiedenen Perspektiven ab, wobei die Figur 4a eine dreidimensionale Draufsicht, die Figur 4b eine dreidimensionale Unteransicht, die Figur 4c einen Frontansicht, die Figur 4d eine Seitenansicht, die Figur 4e eine Unteransicht und die Figur 4f eine Draufsicht des Deckels 5 sind. Der Deckel 5 besitzt in Schnittebenen senkrecht zu der Hochachse 8 des Behälters jeweils die Form eines abgerundeten Rechtecks, so dass sich ein umlaufender Rand 10 des Deckels 5 auch aus vier geraden Abschnitten 20 und vier Bogenabschnitten 21 zusammensetzt. An den beiden langen geraden Abschnitten 20 sind jeweils vier und an den beiden kurzen geraden Abschnitten 20 sind jeweils drei in Umfangsrichtung langgestreckte und gerade ausgebildete Schnapphaken 9 angeordnet. An den vier Bogenabschnitten 21 ist jeweils ein in Umfangsrichtung langgestreckter und entsprechend gebogener Schnapphaken 9' angearbeitet.

Allen Schnapphaken 9, 9' ist gemein, dass sie sich aus einem laschenartigen Zungenabschnitt 22 und einem Hakenabschnitt 23 zusammensetzen, wobei sich der Hakenabschnitt 23 an einem dem Deckel 5 abgewandten Ende des Zungenabschnitts 22 anschließt. Zur besseren Einführung der Schnapphaken 9, 9' in die Rastöffnungen 11, 11' sind die Zungenabschnitte 22 in eine Richtung senkrecht zu der Hochachse 8 des Behälters 1 elastisch verformbar.

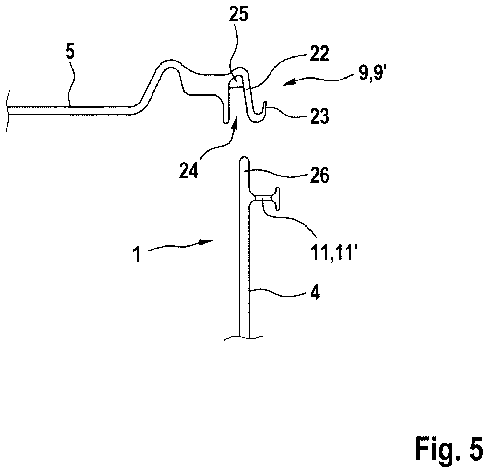

Die Figur 5 zeigt einen Vertikalschnitt durch den Behälter 1 und den Deckel 5 aus Figur 1 im Bereich eines Schnapphakens 9, 9' beziehungsweise einer Rastöffnung 11, 11', wobei Deckel 5 und Behälter 1 nicht zusammengefügt sind. Der Aufbau des Schnapphakens 9, 9' mit seinem Zungenabschnitt 22 und seinem Hakenabschnitt 23 ist deutlich erkennbar. Ferner ist zu sehen, dass der Deckel 5 umlaufend mit einer Dichtnut 24 ausgestattet ist, in der eine Dichtung 25 angeordnet ist.

Der Behälter 1 besitzt an einem dem Boden 3 abgewandten Ende einen umlaufenden Dichtsteg 26, der im vorliegenden Fall durch eine Verlängerung des Mantels 4 des Behälters 1 gebildet wird. Werden Deckel 5 und Behälter 1 zusammengefügt, liegt der Dichtsteg 26 an der Dichtung 25 in der Dichtnut 24 an.

### Bezugszeichenliste:

- 1: Behälter
- 2: Schließstellung
- 3: Boden
- 4: Mantel
- 5: Deckel
- 6: Öffnungsquerschnitt
- 7: Ende des Mantels
- 8: Hochachse
- 9,9': Schnapphaken
- 10: Rand des Deckels
- 11,11': Rastöffnung
- 12: Randflansch
- 13: Tragelasche
- 14: Zwischenräume
- 15: Vertiefung
- 16: Haltegriff
- 17: gerader Abschnitt Randflansch
- 18: Bogenabschnitt Randflansch
- 19: Seite Rastöffnung
- 20: gerader Abschnitt Rand Deckel
- 21: Bogenabschnitt Rand Deckel
- 22: laschenartiger Zungenabschnitt
- 23: Hakenabschnitt
- 24: Dichtnut
- 25: Dichtung
- 26: Dichtsteg

- L: Längsachse Behälter
- Q: Querachse Behälter

## Patentansprüche

1. Behälter (1) mit:
- einem Boden (3),
- einer senkrecht zu dem Boden (3) verlaufenden Hochachse (8),
- einem umlaufend an den Boden (3) anschließenden Mantel (4), der an einem dem Boden (3) abgewandten Ende einen umlaufenden, nach außen vorstehenden Randflansch (12) aufweist,
- einem von dem dem Boden (3) abgewandten Ende (7) des Mantels (4) definierten, einen Zugang zu dem jeweiligen Innenvolumen des Behälters (1) freigebenden Öffnungsquerschnitt (6) und
- einem den Öffnungsquerschnitt (6) in seiner Schließstellung (2) verschließenden Deckel (5), der an Rändern (10) umlaufend mit verteilt und jeweils beabstandet zueinander angeordneten Schnapphaken (9) versehen ist,
wobei in der Schließstellung (2) des Deckels (5) die Schnapphaken (9) in daran angepasste Rastöffnungen (11) in dem Randflansch (12) eingreifen, wodurch ein Formschluss gebildet wird, der eine von dem Boden (3) weg und parallel zu der Hochachse (8) des Behälters (1) gerichtete Bewegung verhindert und wobei der Deckel (5) in der Schließstellung (2) das Innenvolumen des Behälters (1) dicht verschließt, wobei in vier Eckbereichen des Randflanschs (12) jeweils mindestens eine Rastöffnung (11') angeordnet ist, wobei diese Rastöffnungen (11') in der Ebene des Randflanschs (12) betrachtet, zumindest abschnittsweise gekrümmt oder geneigt sowohl zu einer Querachse (Q) und einer Längsachse (L) des Behälters (1) verlaufen oder aus mindestens zwei geraden Abschnitten zusammengesetzt sind, die geneigt zueinander verlaufen und wobei die zugehörigen Schnapphaken (9') daran angepasst ausgebildet sind, wobe: in einer Ebene des Randflanschs (12) betrachtet, langgestreckte Schnapphaken (9') und daran angepasste langgestreckte Rastöffnungen (11') vorjoschun sind, wobei ein dem Boden (3) abgewandtes Ende des Behälters (1) von einem umlaufenden Dichtsteg (26) gebildet wird, **dadurch gekennzeichnet, dass** der Dichtsteg (26) in der Schließstellung (2) des Behälters (1) dichtend an einer Dichtung (25) anliegt, die sich in einer an den Dichtsteg (26) angepassten umlaufenden Dichtnut (24) in dem Deckel befindet, wobei die geformte Rastöffnung en (11') bewichen, dass der Deckel nach Einführen des zugehörigen Schnapphakens (9') in die gekrümmte, geneigte oder aus zwei Abschnitten zusammengesetzte Rastöffnung (11') nicht mehr in Richtung der Längsachse (L) oder Querachse (Q) verschoben werden kann, so dass erreicht wird, dass die übrigen Schnapphaken (9) ausschließlich in die korrespondierenden Rastöffnunge (11) gelangen können, wobei die mehreren "nicht-geraden" Schnapphaken (9') somit eine Führungsfunktion für das ordnungsgemäße Ausrichten und Einsetzen der übrigen, parallel zu der Längs-(L) oder Querachse (Q)angeordneten Schnapphaken (9) übernchmen.

2. Behälter nach Anspruch 1, **dadurch gekennzeichnet, dass** der Mantel (4) in zueinander parallelen Schnittebenen, die jeweils senkrecht zu der Hochachse (8) des Behälters (1) verlaufen, jeweils die Form eines abgerundeten Rechtecks besitzt, wobei vorzugsweise auch der Randflansch (12) die Form eines abgerundeten Rechtecks besitzt und eine Mehrzahl von Rastöffnungen (9, 9') in paarweise gegenüberliegenden gerade verlaufenden Abschnitten (17) des Randflanschs (12) angeordnet sind und mindestens jeweils eine Rastöffnung (11') in sämtlichen vier Bogenabschnitten (18) des Randflanschs (12) angeordnet ist, wobei die Bogenabschnitte (18) jeweils zwei im rechten Winkel zueinander angeordnete geraden Abschnitte (17) miteinander verbinden.

3. Behälter nach Anspruch 1 oder 2, **dadurch gekennzeichnet, dass** die Schnapphaken (9, 9') jeweils aus einem dem Deckel (5) zugewandten laschenartigen Zungenabschnitt (22) und einem Hakenabschnitt (23) zusammengesetzt sind, der jeweils an ein dem Deckel (5) abgewandtes Ende des Zungenabschnitts (22) anschließt, wobei vorzugsweise die Zungenabschnitte (22) in eine Richtung senkrecht zu der Hochachse (8) des Behälters (1) elastisch verformbar sind.

4. Behälter nach mindestens einem der Ansprüche 1 bis 3, **dadurch gekennzeichnet, dass** die Rastöffnungen (11, 11') jeweils auf ihren der Hochachse (8) des Behälters (1) zugewandten Seiten (19) einen Abstand zu dem Mantel (4) aufweisen, der kleiner als 5 mm, vorzugsweise kleiner als 2 mm, weiter vorzugsweise kleiner als 1 mm, noch weiter vorzugsweise gleich Null ist.

5. Behälter nach mindestens einem der vorhergehenden Ansprüche, **dadurch gekennzeichnet, dass** an sich gegenüberliegenden kurzen Seiten der Behälter (1) an einer Oberseite jeweils mit einer Traglasche (13) ausgestattet ist, wobei der Behälter (1) an seinen kurzen Seitenflächen eine vertikal verlaufende Einbuchtung (14) aufweist, deren Breite an der Oberseite des Behälters (1) in etwa der Länge der Traglasche (13) entspricht, wohingegen deren Breite am Boden (3) des Behälters (1) größer ist, wodurch zwei sich gegenüberliegende Rippen (15) der Einbuchtung (14) schräg zueinander verlaufen.

## Claims

1. Container (1) having:
- a base (3);
- a vertical axis (8) which runs so as to be perpendicular to the base (3);
- a jacket (4) which adjoins the base (3) in an encircling manner and which at one end that faces away from the base (3) has an encircling and outwardly projecting peripheral flange (12);
- an opening cross section (6) which is defined by that end (7) of the jacket (4) that faces away from the base (3) and which permits access to the respective internal volume of the container (1); and
- a cover (5) which in the closing position (2) thereof closes the opening cross section (6) and which on peripheries (10) is provided in an encircling manner with snap-fit hooks (9) which are disposed so as to be distributed and each mutually spaced from the other;
wherein the snap-fit hooks (9) in the closing position (2) of the cover (5) engage in latching openings (11) in the peripheral flange (12), which are adapted to said snap-fit hooks (9), on account of which a form-fit which prevents movement which is directed away from the base (3) and parallel with the vertical axis (8) of the container (1) is formed, and wherein the cover (5) in the closing position (2) tightly closes the internal volume of the container (1), wherein at least one latching opening (11') is disposed in each of four corner regions of the peripheral flange (12), wherein these latching openings (11') when viewed in the plane of the peripheral flange (12) at least in portions run so as to be curved or inclined in relation to both a transverse axis (Q) and a longitudinal axis (L) of the container (1), or are assembled from at least two straight portions which run so as to be mutually inclined, and wherein the associated snap-fit hooks (9') are configured so as to be adapted thereto, wherein, when viewed in a plane of the peripheral flange (12), elongate snap-fit hooks (9') and elongate latching openings (11') adapted to the latter are provided, wherein an end of the container (1) that faces away from the base (3) is formed by an encircling sealing web (26), **characterized in that** the sealing web (26) in the closing position (2) of the container (1) bears in a sealing manner on a seal (25) which in the cover is located in an encircling sealing groove (24) that is adapted to the sealing web (26), wherein the shaped latching openings (11') have the effect that the cover, upon introduction of the associated snap-fit hook (9') into the latching opening (11') which is curved, inclined, or assembled from two portions, can no longer be displaced in the direction of the longitudinal axis (L) or transverse axis (Q) such that it is achieved that the remaining snap-fit hooks (9) can make their way exclusively into the corresponding latching openings (11), wherein the plurality of "non straight" snap-fit hooks (9') thus assume a guiding function for the orderly alignment and insertion of the remaining snap-fit hooks (9) which are disposed so as to be parallel to the longitudinal axis (L) or transverse axis (Q).

2. Container according to Claim 1, **characterized in that** the jacket (4) has in mutually parallel sectional planes which each run so as to be perpendicular to the vertical axis (8) of the container (1) in each case the shape of a rounded rectangle, wherein also the peripheral flange (12) preferably has the shape of a rounded rectangle and a plurality of latching openings (9, 9') are disposed in portions (17) of the peripheral flange (12) that are mutually opposite in pairs and run in a straight line, and at least one latching opening (11') is disposed in each of all four arcuate portions (18) of the peripheral flange (12), wherein the arcuate portions (18) each interconnect two straight portions (17) which are disposed so as to be mutually orthogonal.

3. Container as claimed in Claim 1 or 2, **characterized in that** the snap-fit hooks (9, 9') are each assembled from a lug-type tongue portion (22) which faces the cover (5) and from a hook portion (23) in each case adjoining an end of the tongue portion (22) that faces away from the cover (5), wherein the tongue portions (22) are preferably elastically deformable in a direction which is perpendicular to the vertical axis (8) of the container (1).

4. Container as claimed in at least one of Claims 1 to 3, **characterized in that** the latching openings (11, 11') each on that side (19) thereof that faces the vertical axis (8) of the container (1) have a spacing from the jacket (4) that is smaller than 5 mm, preferably smaller than 2 mm, further preferably smaller than 1 mm, even further preferably equal to zero.

5. Container as claimed in at least one of the preceding claims, **characterized in that** the container (1) on mutually opposite short sides, is equipped with a carrying lug (13) on an upper side, wherein the container (1) on the short lateral faces thereof has a vertically running inward bulge (14), the width of which on the upper side of the container (1) approximately corresponds to a length of the carrying lug (13), whereas the width of said inward bulge (14) on the base (3) of the container (1) is larger, on account of which two mutually opposite ribs (15) of the inward bulge (14) run so as to be mutually oblique.

## Revendications

1. Récipient (1), comprenant :
- un fond (3),
- un axe vertical (8) s'étendant perpendiculairement au fond (3),
- une enveloppe (4) se raccordant au fond (3) sur la périphérie, qui présente, au niveau d'une extrémité opposée au fond (3), une bride de bord périphérique (12) saillant vers l'extérieur,
- une section transversale d'ouverture (6) définie par l'extrémité (7) de l'enveloppe (4) opposée au fond (3), ouvrant un accès au volume intérieur respectif du récipient (1) et
- un couvercle (5) fermant la section transversale d'ouverture (6) dans sa position de fermeture (2), qui est pourvu, tout autour au niveau de ses bords (10), de crochets d'encliquetage (9) disposés de manière répartie et espacée à chaque fois les uns des autres,
les crochets d'encliquetage (9), dans la position de fermeture (2) du couvercle (5), s'engageant dans des ouvertures d'encliquetage (11) adaptées à ceux-ci dans la bride de bord (12), de sorte que l'on obtienne un engagement par correspondance de formes qui empêche un mouvement à l'écart du fond (3) et orienté parallèlement à l'axe vertical (8) du récipient (1), le couvercle (5), dans la position de fermeture (2), fermant hermétiquement le volume intérieur du récipient (1), à chaque fois au moins une ouverture d'encliquetage (11') étant disposée dans quatre régions de coin de la bride de bord (12), ces ouvertures d'encliquetage (11'), vu dans le plan de la bride de bord (12), s'étendant au moins en partie sous forme courbe ou inclinée à la fois vers un axe transversal (Q) et un axe longitudinal (L) du récipient (1) ou étant constituées d'au moins deux portions droites qui s'étendent de manière inclinée l'une vers l'autre, et les crochets d'encliquetage associés (9') étant réalisés de manière adaptée à celles-ci, des crochets d'encliquetage allongés (9'), vu dans un plan de la bride de bord (12), et des ouvertures d'encliquetage (11') allongées adaptées à ceux-ci, étant prévus, une extrémité du récipient (1) opposée au fond (3) étant formée par une nervure d'étanchéité périphérique (26),
**caractérisé en ce que** la nervure d'étanchéité (26), dans la position de fermeture (2) du récipient (1), s'applique hermétiquement contre un joint d'étanchéité (25) qui se trouve dans une rainure d'étanchéité périphérique (24) dans le couvercle, adaptée à la nervure d'étanchéité (26), les ouvertures d'encliquetage façonnées (11') faisant en sorte que le couvercle, après l'introduction du crochet d'encliquetage associé (9') dans l'ouverture d'encliquetage (11') courbe, inclinée ou constituée de deux portions, ne puisse plus être déplacé dans la direction de l'axe longitudinal (L) ou de l'axe transversal (Q), de manière à ce que l'on obtienne que les crochets d'encliquetage restants (9) puissent parvenir exclusivement dans les ouvertures d'encliquetage correspondantes (11), la pluralité de crochets d'encliquetage (9') « non droits » assurant ainsi une fonction de guidage pour l'orientation et l'insertion correcte des autres crochets d'encliquetage (9) disposés parallèlement à l'axe longitudinal (L) ou à l'axe transversal (Q).

2. Récipient selon la revendication 1, **caractérisé en ce que** l'enveloppe (4), dans des plans de coupe mutuellement parallèles qui s'étendent à chaque fois perpendiculairement à l'axe vertical (8) du récipient (1), présente à chaque fois la forme d'un rectangle arrondi, la bride de bord (12) présentant de préférence également la forme d'un rectangle arrondi et une pluralité d'ouvertures d'encliquetage (9, 9') étant disposées dans des portions (17) de la bride de bord (12) s'étendant en ligne droite et opposées par paires, et au moins une ouverture d'encliquetage respective (11') étant disposée dans toutes les quatre portions courbes (18) de la bride de bord (12), les portions courbes (18) reliant entre elles à chaque fois deux portions droites (17) disposées à angle droit l'une par rapport à l'autre.

3. Récipient selon la revendication 1 ou 2, **caractérisé en ce que** les crochets d'encliquetage (9, 9') sont constitués à chaque fois d'une portion de langue (22) de type patte tournée vers le couvercle (5) et d'une portion de crochet (23) qui se raccorde à chaque fois à une extrémité de la portion de langue (22) opposée au couvercle (5), les portions de langue (22) étant de préférence déformables élastiquement dans une direction perpendiculaire à l'axe vertical (8) du récipient (1).

4. Récipient selon au moins l'une quelconque des revendications 1 à 3, **caractérisé en ce que** les ouvertures d'encliquetage (11, 11') présentent à chaque fois sur leurs côtés (19) tournés vers l'axe vertical (8) du récipient (1), une distance à l'enveloppe (4) qui est inférieure à 5 mm, de préférence inférieure à 2 mm, plus préférablement inférieure à 1 mm, encore plus préférablement égale à 0.

5. Récipient selon au moins l'une quelconque des revendications précédentes, **caractérisé en ce que** le récipient (1), au niveau de côtés courts opposés, est muni à chaque fois d'une patte de support (13) au niveau d'un côté supérieur, le récipient (1) présentant, au niveau de ses faces latérales courtes, un renfoncement (14) s'étendant verticalement dont la largeur au niveau du côté supérieur du récipient (1) correspond approximativement à la longueur de la patte de support (13), tandis que sa largeur au niveau du fond (3) du récipient (1) est plus grande, de sorte que deux nervures opposées (15) du renfoncement (14) s'étendent obliquement l'une par rapport à l'autre.
